# EUROPEAN PATENT APPLICATION

(11) **EP 4 847 523 A1**
(43) Date of publication of application: **02.12.2026**
(21) Application number: 25893779.6
(22) Date of filing: 25.09.2025
(51) Int. Cl.: C07K 14/415, C12N 15/29

(54) **PARTHENOGENESIS-INDUCING GENE IN PLANTS AND USE THEREOF**

(30) Priority: 19.11.2024 CN 202411657292; 30.04.2025 CN 202510567713
(71) Applicant: China National Rice Research Institute, Hangzhou, Zhejiang 311401 (CN)
(72) Inventor: WANG, Kejian, Hangzhou, Zhejiang 311401 (CN); CHEN, Wenqiang, Hangzhou, Zhejiang 311401 (CN)
(74) Representative: Patent 42
(86) International application number: PCT/CN2025/123976
(87) International publication number: WO 2026/108396

(57) **Abstract**

The present disclosure relates to the technical field of biotechnology and plant breeding, and particularly to genes having parthenogenesis-inducing capability in plants, and uses thereof in plant breeding, including induction of haploid formation and apomixis. By regulating expression of a gene encoding a protein having parthenogenesis-inducing capability, the present disclosure effectively achieves a transition of an egg cell to an embryonic developmental fate, thereby inducing parthenogenesis. The gene and the corresponding induction method may be applied to related plant breeding processes, including production of haploids and synthetic apomixis.

## Description

### TECHNICAL FIELD

The present disclosure relates to the technical field of biotechnology and plant breeding, and particularly to a gene having parthenogenesis-inducing capability in plants, and uses thereof in plant breeding, including induction of haploid formation and apomixis.

### BACKGROUND

In plants, the transition of an egg cell to an embryonic cell fate is an important field of reproductive biology research. This process plays a critical role in the life cycle of plants, particularly in sexual reproduction of seed plants. A plant egg cell is located within the embryo sac and is an unfertilized haploid cell. When fertilization occurs, the egg cell and a sperm cell, usually derived from a pollen tube, fuse to form a diploid zygote. The zygote subsequently develops into a multicellular embryo through cell division and differentiation. During this process, the cytoplasm and genetic material of the egg cell undergo transformation, thereby enabling the cell to acquire the capability to form a complete plant body. Research on the transition of plant egg cells to embryonic cells not only helps to understand the basic principles of plant reproduction and development, but also has practical significance in agricultural practice. For example, a gene capable of inducing an egg cell to transition to an embryonic cell fate in the absence of fertilization, namely a gene having parthenogenesis-inducing capability, may be applied to induction of haploid plants and to the parthenogenesis-inducing process in apomixis technology.

Haploid plants contain a single set of chromosomes and are derived from maternal or paternal gametes. Compared with normal diploid plants, haploid plants have simplified genetic material and exhibit genome-wide genotypic homozygosity. In agricultural science, haploid technology is particularly valuable because the chromosome number of these haploids can be doubled by natural or artificial methods, thereby forming genetically fully homozygous doubled haploid (DH) lines. Owing to their genetic uniformity, such DH plants are a powerful tool in modern breeding technology, particularly when rapid development of germplasm resources with high genetic stability is desired. Compared with traditional breeding methods such as backcrossing or selfing, haploid breeding technology significantly improves the speed and efficiency of producing homozygous lines and greatly accelerates the development cycle of new varieties.

Although haploids occasionally arise under natural conditions, the frequency is extremely low, limiting the practical application value of this phenomenon. Accordingly, scientists have developed two main haploid production technologies: in vitro and in vivo methods. In vitro methods are mainly carried out under laboratory conditions by culturing cells taken from haploid gametophytes, which can differentiate under certain induction conditions to form haploid embryos and ultimately develop into complete haploid plants. This method has a broad application scope and may use microspores (pollen) or megaspores (ovules) as starting materials, but it is highly dependent on plant genotype, and different plants vary in culture difficulty and success rate. In addition, mutations readily occur and accumulate during in vitro culture, and complex and precise technical operations are required, including selection of anther stage, pretreatment of anthers, and optimization of medium formulation.

**In** vivo methods use natural or specifically technologically improved pollination or induction approaches, including irradiated pollen technology, interspecific hybridization, and pollination using inducer lines. An advantage of this method is that haploid embryos can be induced directly within the plant body and subsequently form haploid seeds and progeny plants, thereby potentially overcoming limitations of in vitro culture, such as low efficiency, complicated operation, and background dependence. Inducer lines in in vivo methods may operate through a unique double-fertilization mechanism. In normal double fertilization, one sperm cell fuses with an egg cell to produce a diploid embryo, while another sperm cell fuses with the central cell to form endosperm. In the use of haploid inducer line technology, haploid embryos can be induced by single fertilization or by genome elimination after double fertilization.

Haploid induction technology is not only used in breeding to rapidly obtain homozygous germplasm resources, but may also be combined with clonal gamete technology to develop apomixis systems. Apomixis technology can fix and utilize heterosis of hybrids without going through a traditional fertilization process, thereby simplifying hybrid breeding procedures and production practices. Application of this technology can significantly reduce labor and material inputs in hybrid production and has important economic and social value. Through these efficient breeding technologies, modern agriculture can better meet the demands of global food security and sustainable development.

### SUMMARY

The present disclosure was completed by analyzing expression patterns of rice genes, screening transcription factor genes that are expressed in sperm cells, not expressed in egg cells, and not expressed in other tissues, discovering a plurality of transcription factors of the RWP-RK family including LOC _Os12g12970, further predicting that genes of this family may play an important role in the transition of egg cell fate to embryonic fate after fertilization, and further exploring and experimentally confirming that RWP-RK transcription factors from multiple species, including LOC_Os12g12970 in rice, all have parthenogenesis-inducing capability.

Accordingly, the present disclosure provides a parthenogenesis-inducing gene. Expression of the gene in an egg cell can achieve transition of an unfertilized egg cell fate to an embryonic fate, thereby inducing generation of a haploid plant. The disclosure provides a new gene resource and application technology for plant haploid induction and apomixis.

The present disclosure provides a protein having parthenogenesis-inducing capability, wherein the protein is a transcription factor of the RWP-RK protein family and comprises the following conserved domains: TITFEDIRKYFHLPI and KEAAKELNVCLTTLKKICREHGIPRWPHRKIKSLNKLIKNI; and wherein the protein comprises a sequence having greater than 60% homology to SEQ ID NO:1 or SEQ ID NO:2, preferably greater than 70%, 80%, or 90% homology, more preferably greater than 95%, 98%, or 99% homology, and still has parthenogenesis-inducing capability.

Specifically, the protein is LOC_Os12g12970 (SEQ ID NO:4), LOC_Os01g14420 (SEQ ID NO:6), LOC_Os04g47640, or LOC _Os12g12970.2 (SEQ ID NO:28) in rice; homologous proteins in maize are GRMZM2G004663 (SEQ ID NO:8) and AC233880 (SEQ ID NO:10); homologous proteins in wheat are TRAES3BF003800040CFD (SEQ ID NO:12) and TraesCS7A03G1206100 (SEQ ID NO:14); homologous proteins in sorghum are Sobic.003G000800 (SEQ ID NO:16) and Sobic.008G082000 (SEQ ID NO:18); a homologous protein in soybean is Glyma.06G198300 (SEQ ID NO:20); a homologous protein in foxtail millet is Seita.3G091700 (SEQ ID NO:22); homologous proteins in tomato are Solyc08g062200 (SEQ ID NO:24) and Solyc12g011190 (SEQ ID NO:26); or the protein comprises a homologous sequence having greater than 22% homology to LOC _Os12g12970, preferably greater than 35% homology, more preferably greater than 45% homology; or comprises a homologous sequence having greater than 42% homology to LOC _Os01g14420, preferably greater than 60% homology, more preferably greater than 70% homology; or comprises a homologous sequence having greater than 60% homology to any of the foregoing proteins, preferably greater than 70%, 80%, or 90% homology, more preferably greater than 95%, 98%, or 99% homology, and still has parthenogenesis-inducing capability.

Preferably, the gene is LOC_Os12g12970 (SEQ ID NO:3), LOC_Os01g14420 (SEQ ID NO:5), LOC_Os04g47640, LOC _Os12g12970.2 (SEQ ID NO:27), or the genomic gene shown in SEQ ID NO:29 in rice; homologous genes in maize are GRMZM2G004663 (SEQ ID NO:7) and AC233880 (SEQ ID NO:9); homologous genes in wheat are TRAES3BF003800040CFD (SEQ ID NO:11) and TraesCS7A03G1206100 (SEQ ID NO:13); homologous genes in sorghum are Sobic.003G000800 (SEQ ID NO:15) and Sobic.008G082000 (SEQ ID NO:17); a homologous gene in soybean is Glyma.06G198300 (SEQ ID NO:19); a homologous gene in foxtail millet is Seita.3G091700 (SEQ ID NO:21); and homologous genes in tomato are Solyc08g062200 (SEQ ID NO:23) and Solyc12g011190 (SEQ ID NO:25).

The present disclosure also provides use of the protein or the encoding gene for inducing parthenogenesis in a plant, specifically for inducing haploids; or use thereof in apomixis.

Specifically, the plant is a monocotyledonous plant or a dicotyledonous plant. The monocotyledonous plant is preferably a plant of Poaceae, including but not limited to rice (*Oryza sativa*)*,* maize *(Zea mays*), wheat (*Triticum aestivum*), barley (*Hordeum vulgare*), rye (*Secale cereale*), oat (*Avena sativa*), sorghum (*Sorghum bicolor*), proso millet (*Panicum miliaceum*), foxtail millet (*Setaria italica*), sugarcane (*Saccharum officinarum*), perennial ryegrass (*Lolium perenne*), annual ryegrass (*Lolium multiflorum*)*,* fescue *(Festuca arundinacea),* wildrye (*Elymus spp*.), crested wheatgrass (*Agropyron cristatum*), bermudagrass (*Cynodon dactylon*), zoysiagrass (*Zoysia japonica*), tall fescue *(Festuca arundinacea),* bromegrass (*Bromus inermis*), sudangrass (*Sorghum sudanense*), and reed (*Phragmites australis*). The dicotyledonous plant preferably includes plants of the following families and genera: Fabaceae, such as soybean (*Glycine max*), peanut (*Arachis hypogaea*)*,* alfalfa (*Medicago sativa*), cowpea (*Vigna unguiculata*), mung bean (*Vigna radiata*), adzuki bean (*Vigna angularis*)*,* chickpea (*Cicer arietinum*), hyacinth bean (*Lablab purpureus*), red clover (*Trifolium pratense*)*,* white clover (*Trifolium repens*), broad bean (*Vicia faba*), and lupin (*Lupinus albus, Lupinus angustifolius*); Brassicaceae, such as rapeseed (*Brassica napus*), Chinese cabbage (*Brassica rapa subsp. pekinensis*), cabbage *(Brassica oleracea* var. *capitata*), radish (*Raphanus sativus*), mustard (*Brassica juncea*), Chinese kale (*Brassica oleracea* var. *alboglabra*), kale *(Brassica oleracea* var. *sabellica*), turnip (*Brassica rapa subsp. rapa),* broccoli *(Brassica oleracea* var. *italica),* and cauliflower (*Brassica oleracea* var. *botrytis*); Solanaceae, such as potato (*Solanum tuberosum*), tomato (*Solanum lycopersicum*), chili pepper (*Capsicum annuum*), eggplant (*Solanum melongena*), tobacco (*Nicotiana tabacum*), goji berry (*Lycium barbarum*), Chinese lantern plant (*Physalis alkekengi*), black nightshade (*Solanum nigrum*), sweet potato (*Ipomoea batatas*), green pepper *(Capsicum annuum),* and bird pepper (C*apsicum frutescens*); Cucurbitaceae, such as cucumber *(Cucumis sativus),* watermelon (*Citrullus lanatus*), melon (*Cucumis melo*), pumpkin (*Cucurbita spp.,* for example *C. pepo, C. maxima,* and C. *moschata),* loofah (*Luffa cylindrica*), bitter melon (*Momordica charantia*), wax gourd (*Benincasa hispida*)*,* bottle gourd *(Lagenaria siceraria),* chayote (*Sechium edule*), and zucchini (*Cucurbita pepo*); Asteraceae, such as sunflower (*Helianthus annuus*), lettuce (*Lactuca sativa*), dandelion (*Taraxacum officinale*)*,* garland chrysanthemum (*Chrysanthemum coronarium*), and asparagus lettuce (*Lactuca sativa* var. *angustana*); Apiaceae, such as carrot (*Daucus carota subsp. sativus),* celery *(Apium graveolens),* coriander/cilantro *(Coriandrum sativum),* fennel (*Foeniculum vulgare*)*,* and dill (*Anethum graveolens*); Vitaceae, such as grape (*Vitis vinifera*), Amur grape *(Vitis amurensis*), fox grape (*Vitis labrusca*), and Eurasian grape (*Vitis vinifera ssp. sativa*); Malvaceae, such as cotton (*Gossypium hirsutum*); Pedaliaceae, such as sesame (*Sesamum indicum*) and African sesame (*Sesamum alatum*); Liliaceae, such as onion (*Allium cepa*), garlic (*Allium sativum*), Chinese chive (*Allium tuberosum*)*,* Welsh onion (*Alliumfistulosum*)*,* lily (*Lilium spp*.), and daylily (*Hemerocallis citrina*); Amaranthaceae, such as spinach (*Spinacia oleracea*), Swiss chard (*Beta vulgaris* var. *cicla*), amaranth (*Amaranthus tricolor*), and *Amaranthus spp.;* Rosaceae, such as strawberry (*Fragaria* × *ananassa*); and plants of other families and genera, such as beet (*Beta vulgaris, Chenopodiaceae*), cassava (*Manihot esculenta, Euphorbiaceae*), coffee (*Coffea arabica, Rubiaceae*)*,* cacao (*Theobroma cacao, Sterculiaceae*), and castor bean (*Ricinus communis, Euphorbiaceae).*

The present disclosure further provides a method for inducing a plant haploid, which is implemented by expressing, in an egg cell, a gene encoding the protein described herein, and subsequently identifying and obtaining haploid progeny from progeny thereof by flow cytometric screening; or by combining the above haploid induction method with a clonal gamete method, such as simultaneously mutating OSD1, PAIR1, and REC8 in rice, and subsequently obtaining clonal progeny plants from progeny thereof.

Specifically, expression of the gene is driven by a heterologous or homologous promoter active in an egg cell.

More specifically, the promoter is selected from AtDD45 (At2g21740), AtEC1.1 (Atlg76750), AtEC1.3 (At2g21750), AtEC1.4 (At4g39340), AtEC1.5 (At5g64720), OsECA1 (LOC _Os03g18530), OsECA2 (LOC _Os11g06730), OsECA3 (LOC _Os12g06970), or another heterologous or homologous promoter having egg cell expression characteristics.

In specific embodiments, the promoter of an endogenous gene encoding the protein is modified by gene editing or mutagenesis so that the endogenous gene is expressed in an egg cell; or an activation system is expressed in an egg cell to induce expression of the endogenous gene in the egg cell, for example, the activation system is a CRISPR/Cas9-based specific gene activation system CRISPRa, such as dCas9-TV, dzCas9-Act3.0, dSpRY-Act3.0, or the like.

Specifically, an encoding sequence of the protein is inserted downstream of an endogenous promoter active in an egg cell to induce expression of the gene in an egg cell; or a genome is modified to achieve large-fragment deletion and chromosomal inversion, so as to recombine the endogenous gene with a promoter on the genome to induce expression of the gene in an egg cell.

In another embodiment, the protein is expressed by transient expression, particularly wherein the expression site is an egg cell. For example, the transient expression is carried out by nanoparticle-mediated delivery of a plasmid.

Optionally, haploid plants are obtained from progeny of the resulting plant material by flow cytometric screening, molecular markers, phenotypic identification, or marker-assisted methods, and subsequently the haploid plants may be subjected to chemical treatment or spontaneous genome doubling events to obtain genotype-homozygous doubled haploid materials for use in plant breeding processes;
in addition, when combined with clonal gamete technology, clonal seeds and plants are obtained from progeny thereof, thereby achieving apomixis in plants, which may further be used for hybrid plant breeding or fixation of plant heterozygous genotypes, and for production of hybrids.

By regulating expression of a gene encoding a protein having parthenogenesis-inducing capability, the present disclosure effectively achieves a transition of an egg cell to an embryonic developmental fate, thereby inducing parthenogenesis. The gene and the corresponding induction method may be applied to related plant breeding processes, including production of haploids and synthetic apomixis.

### BRIEF DESCRIPTION OF THE DRAWINGS

Fig. 1 shows expression patterns of candidate genes in various tissues.
Fig. 2 shows a vector map of pC1300-OsECA::LOC_Os12g12970.
Fig. 3 shows plant phenotypes of pC1300-OsECA::LOC_Os12g12970 transgenic lines.
Fig. 4 shows that ectopic expression of LOC_Os12g12970 in an egg cell induces autonomous development of the egg cell into an embryo-like structure.
Fig. 5 shows that ectopic expression of LOC_Os12g12970 in an egg cell induces production of haploid progeny.
Fig. 6 shows a phylogenetic tree of RWP-RK family genes.
Fig. 7 shows that the RWP-RK family has two major conserved structural regions.
Fig. 8 shows a comparison of sequence similarity among proteins in the candidate branch. The values shown in the figure are corresponding similarity percentages.
Fig. 9 shows vector maps for ectopic egg cell expression of homologous genes from various species.
Fig. 10 shows examples of flow cytometric detection results of progeny after ectopic egg cell expression of homologous genes from various species.
Fig. 11 shows a vector map of pC1300-MiMe-OsECA::LOC_Os12g12970.
Fig. 12 shows genotypes of MiMe-OsECA::LOC_Os12g12970 lines.
Fig. 13 shows plant phenotypes of MiMe-OsECA::LOC_Os12g12970.
Fig. 14 shows flow cytometric analysis of progeny of MiMe-OsECA::LOC_Os12g12970.
Fig. 15 shows whole-genome sequencing analysis of clonal diploid progeny of MiMe-OsECA::LOC _Os12g12970.
Fig. 16 shows positions of editing target sites in the promoter region and terminator region of LOC_Os12g1297.
Fig. 17 shows flow cytometric detection results of progeny after editing of the promoter region and terminator region of LOC_Os12g1297.
Fig. 18 shows comparison of protein sequences corresponding to LOC_Os12g12970 transcripts and gene exon structures.
Fig. 19 shows vector structure diagrams of OsECA::LOC _Os12g12970.2 (left) and OsECA::LOC _Os12g12970-gDNA (right).
Fig. 20 shows a representative flow cytometric diagram of haploid identification results of progeny after transient expression of LOC_Os12g12970.

### DETAILED DESCRIPTION OF THE EMBODIMENTS

### Example 1: Ectopic expression of LOC_Os12g12970 in an egg cell induces development of the egg cell toward an embryonic fate and produces haploid progeny.

### 1. Screening of candidate genes

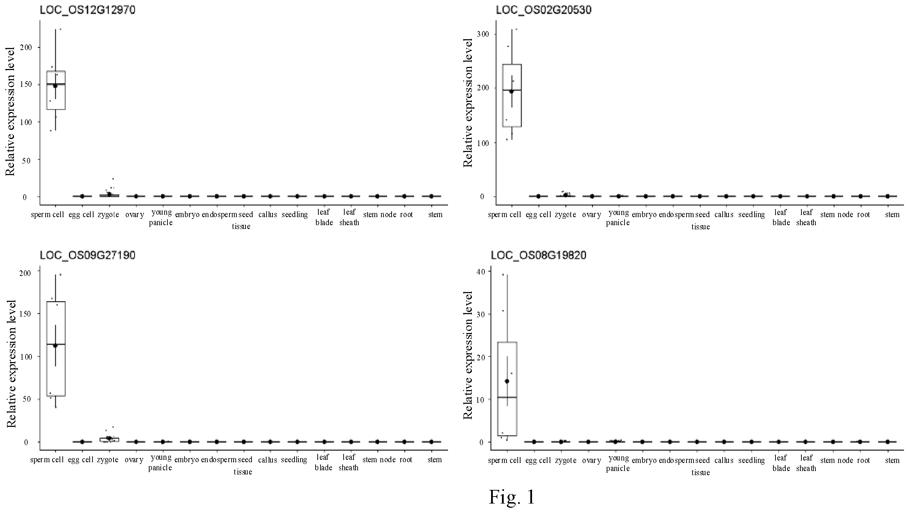

Before double fertilization in angiosperms, an egg cell maintains its cellular state and does not enter an embryonic development process. After fusion with a sperm cell, however, the egg cell initiates embryonic development. During this fusion process, a related embryonic development initiation signal may be introduced by the sperm cell, thereby initiating the transition of the egg cell fate to an embryonic developmental fate. Key genes regulating this transition should have the following characteristics: 1) expression in sperm cells before fertilization; 2) no expression in egg cells before fertilization; 3) possible expression to a certain extent in the zygote after fertilization (early embryo); 4) in view of the ability of the target gene to determine cell fate, the gene should also not be expressed or should be expressed at a low level in tissues other than the above cells; and 5) the gene most likely encodes a transcription factor so that it can rapidly initiate expression of related genes after fusion of the sperm cell and egg cell. Based on the above characteristics, expression pattern analysis was performed on rice genes to screen transcription factor genes that are expressed in sperm cells, not expressed in egg cells, and not expressed in other tissues. These include multiple transcription factors of the RWP-RK family: LOC _Os12g12970, LOC_Os02g20530, LOC_Os09g27190, and LOC_Os08g19820 (Fig. 1). It was therefore speculated that genes of this family may play an important role in the transition of egg cell fate to embryonic fate after fertilization. Further, in view of other information and evolutionary relationships, five genes in this family were selected for experiments, and the results showed that LOC _Os12g12970, LOC_Os01g14420, and LOC **_**Os04g47640 had effects.

### 2. LOC_Os12g12970 can promote the transition of an unfertilized egg cell to an embryonic developmental fate.

### 1) Vector construction

To determine whether these candidate genes have the capability to initiate the transition of egg cell fate to an embryonic developmental fate, an experiment was designed in which an egg cell expression promoter OsECA in rice was used to drive specific expression of LOC_Os12g12970 in egg cells. The amplification primers used for OsECA were:
adPmeI-OsECA1-F: aaacactgatagtttTATACATGGGAGTCTAGTGCAATATTACTC,
OsECA1-R: GGTTTTTCTTTCTAGCTTTGCTGCTTGG;
the amplification primers used for LOC_Os12g12970 were:
F: CTAGAAAGAAAAACCATGGCGGGCGACGGCGGCAAC;
R: AATGTTTGAACGATCTTATTGATCAAGACCAGCAACTATCCTTC.

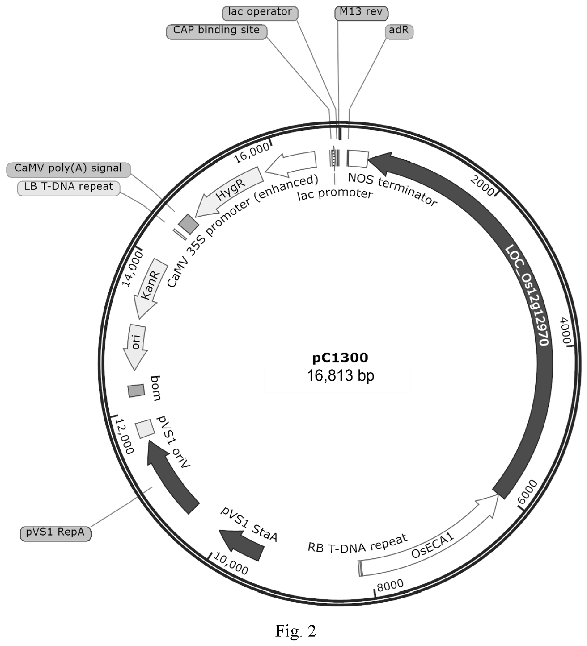

Finally, the expression cassette was ligated to the PmeI site of the pC1300 vector by Gibson assembly, thereby obtaining pC1300-OsECA::LOC_Os12g12970, the vector map of which is shown in Fig. 2.

### 2) Obtaining pC1300-OsECA::LOC_Os12g12970 transgenic plants

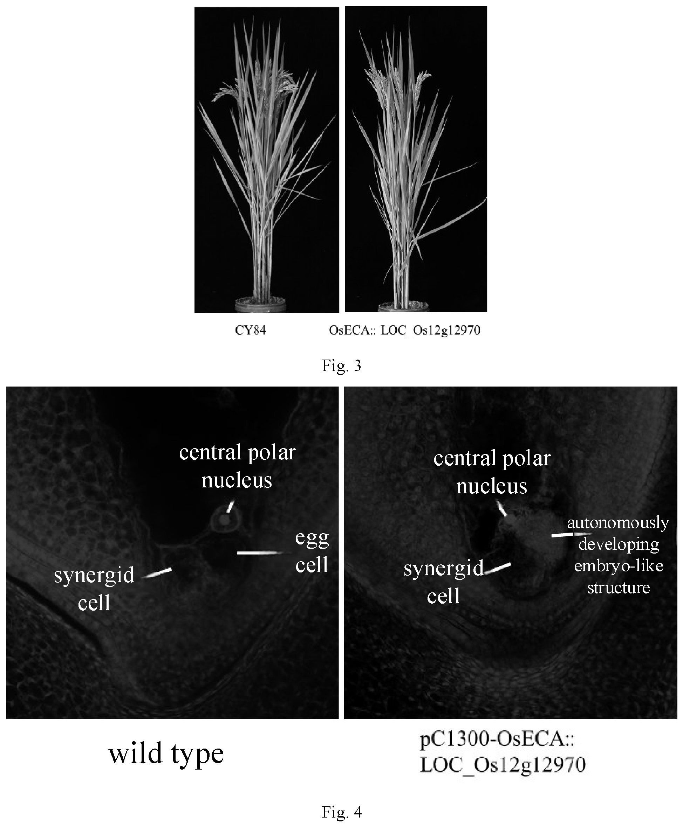

The expression vector pC1300-OsECA::LOC_Os12g12970 was introduced into Agrobacterium tumefaciens strain EHA105 by electroporation, and this binary expression vector was introduced into calli of rice Chunyou 84 by an Agrobacterium-mediated method. The specific transformation method was as follows: embryos of hybrid rice Chunyou 84 seeds were sterilized and then inoculated onto a medium for inducing callus tissue. After culture for 1 week, embryogenic calli that were vigorous, light yellow in color, and relatively loose were selected as transformation recipients. Rice calli were respectively infected with the EHA105 strain containing the pC1300-OsECA::LOC_Os12g12970 plasmid, cultured in the dark at 25°C for 3 days, and then resistant calli and transgenic plants were screened on a selection medium containing 50 mg/L hygromycin. Transgenic plants growing normally on the hygromycin selection medium were selected. PCR detection was performed on the obtained lines to determine whether they contained the corresponding transgenic fragment, and lines in which bands were detected were lines containing the target transgenic component. Compared with the wild type, pC1300-OsECA::LOC_Os12g12970 transgenic lines had similar plant height and tiller number, but their seed setting rate decreased (Fig. 3).

### 3) Autonomous development of egg cells in pC1300-OsECA::LOC_Os12g12970 transgenic lines

To determine whether ectopic expression of LOC_Os12g12970 in egg cells can achieve transition of egg cells to an embryonic developmental fate in the absence of fertilization, wild-type and pC1300-OsECA::LOC_Os12g12970 materials were emasculated before flowering, and internal cell structures in the emasculated ovaries were observed. It was found that egg cells of the wild type still maintained a single-cell state, whereas egg cells in pC1300-OsECA::LOC _Os12g12970 autonomously divided and formed embryo-like structures (Fig. 4). This indicates that expression of the LOC_Os12g12970 gene in egg cells can induce the transition of egg cells to an embryonic developmental fate.

### 4) Selfed progeny of pC1300-OsECA::LOC_Os12g12970 transgenic lines can induce production of haploids

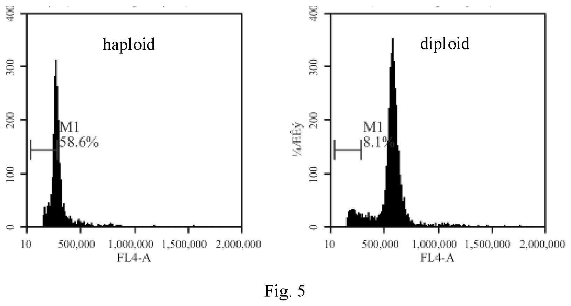

To determine whether expression of LOC_Os12g12970 in egg cells can induce production of haploid progeny, progeny produced by selfing pC1300-OsECA::LOC_Os12g12970 were subjected to flow cytometric analysis to detect plant ploidy. The specific analysis process was as follows: a rice leaf segment about 0.5 cm in length was taken and chopped into a homogenate with a double-edged blade in 1 mL of ice-precooled LBO1 buffer; the LBO1 buffer components were as follows: 15 mM Tris, 2 mM disodium EDTA, 0.5 mM sperminetetrahydrochloride, 80 mM KCl, 20 mM NaCl, 0.1% (v/v) Triton X-100, 15 mM β-mercaptoethanol, and pH 7.5; the homogenate was then filtered through a 45 µm filter to remove leaf debris; nuclei were collected by centrifugation at 1200 rcf and 4°C for 5 minutes, and stained for 20 minutes with 500 µL of LBO1 buffer containing 25 mg/L propidium iodide and 25 mg/L DNase-free RNase A; finally, nuclear ploidy was analyzed on a flow cytometer. The obtained results are shown in Fig. 5, wherein the peak of the diploid control was at about 70,000 (diploid), whereas the detected haploid peak was at 35,000 (haploid).

By the above method, a total of 5 haploid progeny were screened from 155 OsECA::LOC_Os12g12970 progeny plants, and the haploid induction rate was about 3.2%. This indicates that ectopic expression of LOC_Os12g12970 in egg cells can induce production of haploids.

### Example 2: Screening of homologous genes from multiple species

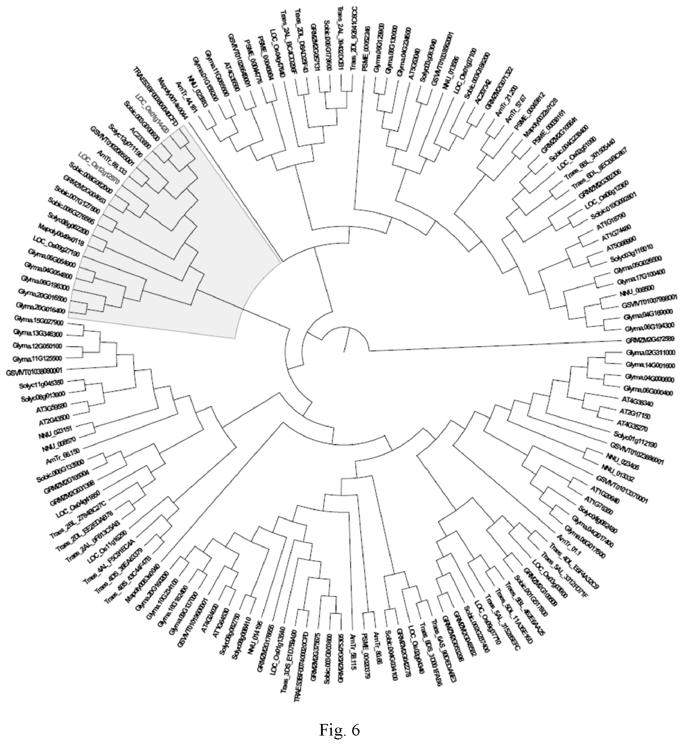

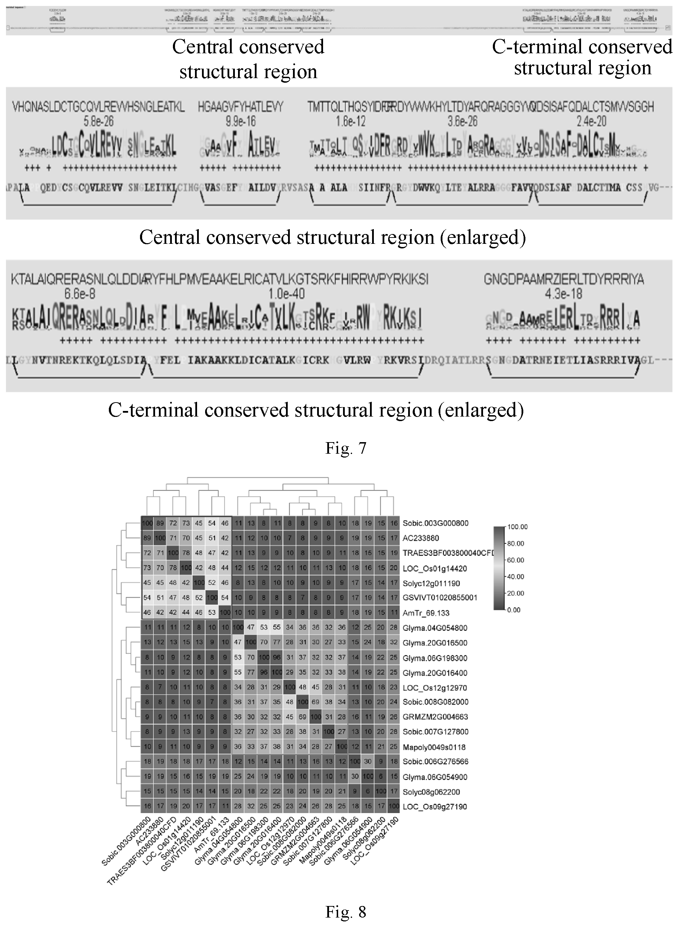

To screen whether other genes in the same family as LOC_Os12g12970 also have haploid induction capability, a phylogenetic similarity tree analysis was performed on members of the RWP-RK family in multiple species. The results in Fig. 6 show that, in rice, two other genes are located in the same branch as LOC _Os12g12970, namely LOC_Os01g14420 and LOC_Os09g27190; genes in maize located in this branch include GRMZM2G004663 and AC233880; genes in wheat located in this branch include TRAES3BF003800040CFD and TraesCS7A03G1206100; genes in sorghum located in this branch include Sobic.003G000800, Sobic.008G082000, Sobic.007G127800, and Sobic.006G276566; genes in tomato located in this branch include Solyc12g011190 and Solyc08g062200; a gene in grape located in this branch is GSVIVT01020855001; and genes in soybean located in this branch include Glyma.06G054900, Glyma.04G054800, Glyma.06G198300, Glyma.20G016400, and Glyma.20G016500. In addition, proteins of this family mainly have two major protein structural regions (Fig. 7), and the sequences of these two regions in LOC_Os12g12970 are SEQ ID NO:1 and SEQ ID NO:2, respectively, indicating that these two regions may play an important role in their function. Overall, the above indicates that LOC_Os12g12970 has relatively similar homologous genes in multiple species. The sequence similarity among proteins within the branch is not high, but two major similar regions are still present, namely region 1 having greater than 42% similarity to rice LOC _Os01g14420 and region 2 having greater than 28% similarity to LOC_Os12g12970 (Fig. 8).

### Example 3

Therefore, to verify whether candidate genes in various species having greater than 42% similarity to rice LOC _Os01g14420 and greater than 28% similarity to LOC_Os12g12970, as well as other genes in the same rice family, still have haploid induction capability, experiments were designed in which an egg cell promoter was used to drive expression of candidate genes from various species in rice for testing.

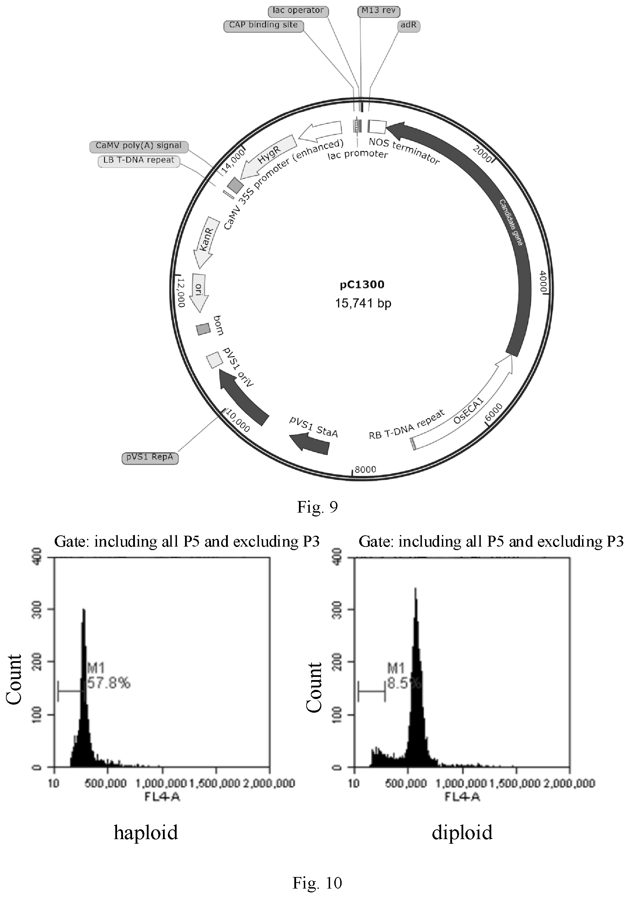

Finally, each expression cassette was ligated to the PmeI site of the pC1300 vector by Gibson assembly, thereby obtaining an ectopic egg cell expression vector for each gene, the vector maps of which are shown in Fig. 9.

Transgenic lines ectopically expressing homologous genes from various species in egg cells were obtained by the same method as described above, and ploidy of their progeny was detected by flow cytometry. Specifically, the expression vector was introduced into *Agrobacterium tumefaciens* strain EHA105 by electroporation, and this binary expression vector was introduced into calli of rice Chunyou 84 by an Agrobacterium-mediated method. The specific transformation method was as follows: embryos of hybrid rice Chunyou 84 seeds were sterilized and then inoculated onto a medium for inducing callus tissue. After culture for 1 week, embryogenic calli that were vigorous, light yellow in color, and relatively loose were selected as transformation recipients. Rice calli were respectively infected with EHA105 strains containing target plasmids, cultured in the dark at 25°C for 3 days, and then resistant calli and transgenic plants were screened on a selection medium containing 50 mg/L hygromycin. Transgenic plants growing normally on the hygromycin selection medium were selected. PCR detection was performed on the obtained lines to determine whether they contained the corresponding transgenic fragment, and lines in which bands were detected were lines containing the target transgenic component.

To determine whether expression of homologous genes from various species in egg cells can induce production of haploid progeny, progeny produced by selfing each transgenic line were subjected to flow cytometric analysis to detect plant ploidy. The specific analysis process was as follows: a rice leaf segment about 0.5 cm in length was taken and chopped into a homogenate with a double-edged blade in 1 mL of ice-precooled LBO1 buffer; the LBO1 buffer components were as follows: 15 mM Tris, 2 mM disodium EDTA, 0.5 mM sperminetetrahydrochloride, 80 mM KCl, 20 mM NaCl, 0.1% (v/v) Triton X-100, 15 mM β-mercaptoethanol, and pH 7.5; the homogenate was then filtered through a 45 µm filter to remove leaf debris; nuclei were collected by centrifugation at 1200 rcf and 4°C for 5 minutes, and stained for 20 minutes with 500 µL of LBO1 buffer containing 25 mg/L propidium iodide and 25 mg/L DNase-free RNase A; finally, nuclear ploidy was analyzed on a flow cytometer. The obtained results are shown in Fig. 10, wherein the peak of the diploid control was at about 70,000 (diploid), whereas the detected haploid peak was at 35,000 (haploid).

By the above method, haploid identification was performed on progeny ectopically expressing homologous genes from various species in egg cells, and the genes having haploid induction capability are shown in Table 2. This indicates that homologous genes of this class of genes in multiple species still have haploid induction capability. The similarity features of the proteins encoded by these genes are that they have at least 42% similarity to rice LOC_Os01g14420 or at least 22% similarity to LOC_Os12g12970 (Table 1).

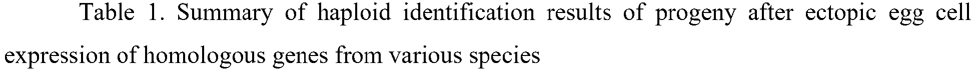

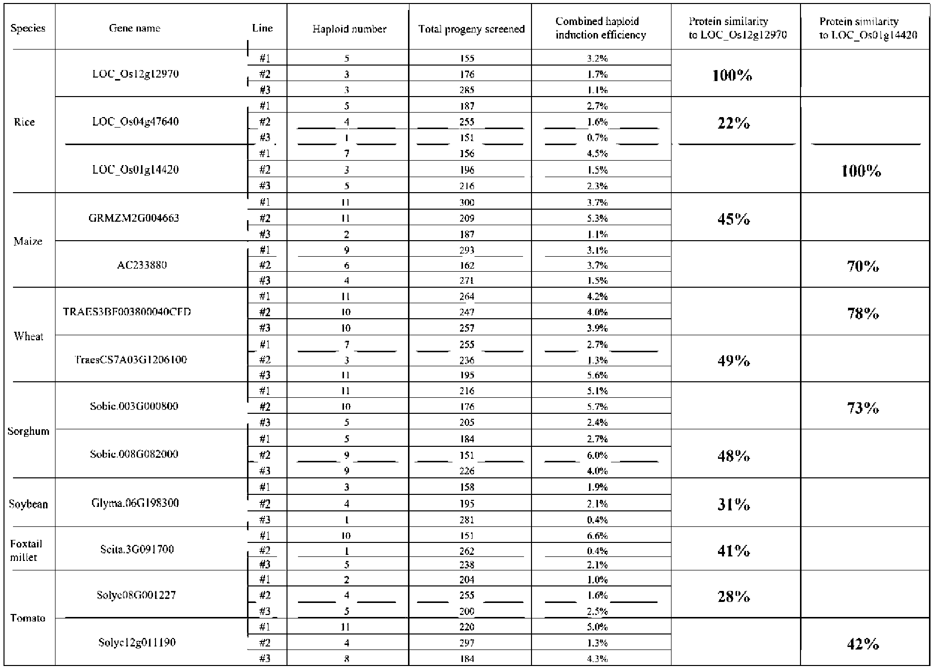

### Example 4: Ectopic expression of LOC_Os12g12970 in an egg cell combined with a clonal gamete MiMe mutant to induce production of clonal progeny

Apomixis can be used to fix heterosis and achieve self-propagation of hybrids, thereby saving a large amount of hybrid rice breeding cost. Currently reported artificially synthesized apomixis systems all combine a clonal gamete method, such as MiMe, with a haploid induction method. To determine the effect of the haploid induction gene of the present disclosure in an apomixis system, an experiment was designed in which LOC_Os12g12970 was ectopically expressed in egg cells while the three rice genes OsOSD1, OsPAIR1, and OsREC8 were knocked out.
1) Construction of a vector for gene-editing knockout of OsOSD1, OsPAIR1, and OsREC8

The following sites were selected as target sites for knocking out OSD1 (LOC_Os02g37850), PAIR1 (LOC_Os03g01590), and REC8 (LOC_Os05g50410) by a CRISPR-Cas9 gene editing system, wherein the underlined sequence indicates the PAM sequence:
OSD1-1 gene knockout site: CTGCCGCCGACGAGCAACAAGG; OSD1-2 gene knockout site: CCGCCGGGAGCCGTGGCGGTCAA.
PAIR1 gene knockout site: AAGCAACCCAGTGCACCGCTGG; REC8 gene knockout site: CCCATGGCACTAAGGCTCTCCG.

Two complementary DNA sequences were designed respectively as follows: GGCA was added before the forward sequence, and AAAC was added before the reverse complementary sequence.

SK-gRNA has two AarI restriction sites. After digestion with AarI, a vector having sticky ends was formed. After denaturation and annealing of the forward and reverse primers of the designed target sequence, the target sequence was ligated into the previously constructed intermediate vector SK-gRNA using T4 ligase to form a single target gRNA.

### 2) Tandem assembly of multiple gRNAs and construction of the final binary expression vector

Taking advantage of the fact that BglII and BamHI, NheI and XbaI, and SalI and XhoI are isocaudomers, gRNAs were assembled onto the pC1300-Cas9 vector to obtain a multi-gene knockout vector capable of simultaneously knocking out the three genes REC8, OSD1, and PAIR1, namely pC1300-Cas9-gRNA1 OSD1-gRNA2OSD1-gRNA REC8-gRNA PAIR1 (hereinafter referred to as pC1300-MiMe), which was used as a backbone vector for subsequent construction (MiMe backbone vector).

Subsequently, the amplification primers for OsECA were used: adPmeI-OsECA1-F: aaacactgatagtttTATACATGGGAGTCTAGTGCAATATTACTC, OsECA1-R: GGTTTTTCTTTCTAGCTTTGCTGCTTGG; and the amplification primers for LOC_Os12g12970 were used: V-F: CTAGAAAGAAAAACCATGGCGGGCGACGGCGGCAAC; V-R: AATGTTTGAACGATCTTATTGATCAAGACCAGCAACTATCCTTC.

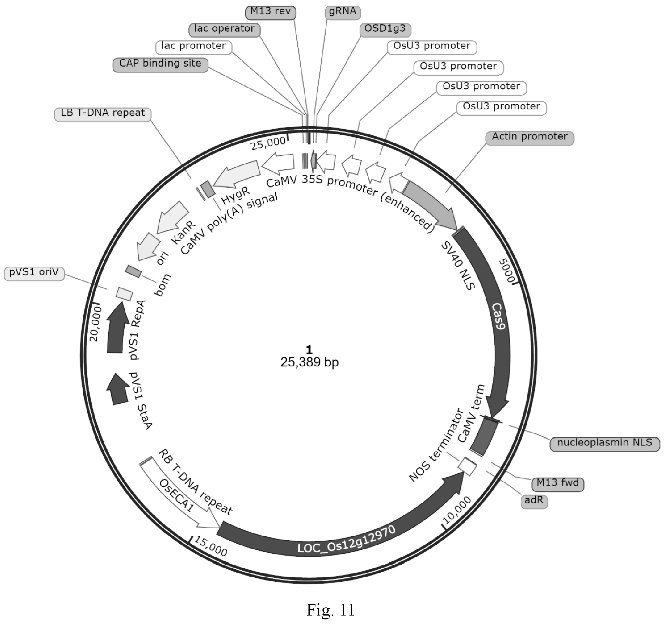

Finally, the expression cassette was ligated to the PmeI site of the pC1300-MiMe vector by Gibson assembly, thereby obtaining pC1300-MiMe-OsECA::LOC_Os12g12970, the vector map of which is shown in Fig. 11.

### 3) Obtaining pC1300-MiMe-OsECA::LOC_Os12g12970 transgenic plants

The expression vector pC1300-MiMe-OsECA::LOC_Os12g12970 was introduced into Agrobacterium tumefaciens strain EHA105 by electroporation, and this binary expression vector was introduced into calli of rice Chunyou 84 by an Agrobacterium-mediated method. The specific transformation method was as follows: embryos of hybrid rice Chunyou 84 seeds were sterilized and then inoculated onto a medium for inducing callus tissue. After culture for 1 week, embryogenic calli that were vigorous, light yellow in color, and relatively loose were selected as transformation recipients. Rice calli were respectively infected with the EHA105 strain containing the pC1300-MiMe-OsECA::LOC_Os12g12970 plasmid, cultured in the dark at 25°C for 3 days, and then resistant calli and transgenic plants were screened on a selection medium containing 50 mg/L hygromycin. Transgenic plants growing normally on the hygromycin selection medium were selected. PCR detection was performed on the obtained lines to detect editing effects of the three MiMe genes. The detection primers used were as follows:
REC8-F: gcgacgcttcactcgaagatca; REC8-R: cgccatgcctcgttgatctcaa
OSD1-F: atctccaggatgcctgaagtgag; OSD1-R: cctagactgctactcttgctagtgat
PAIR1-F: ctgtacctgtgcatctaattacag; PAIR1-R: ccccatcttatgtactgagcttgccag.

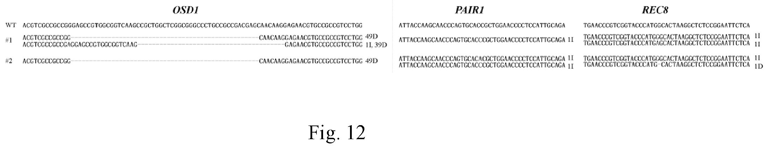

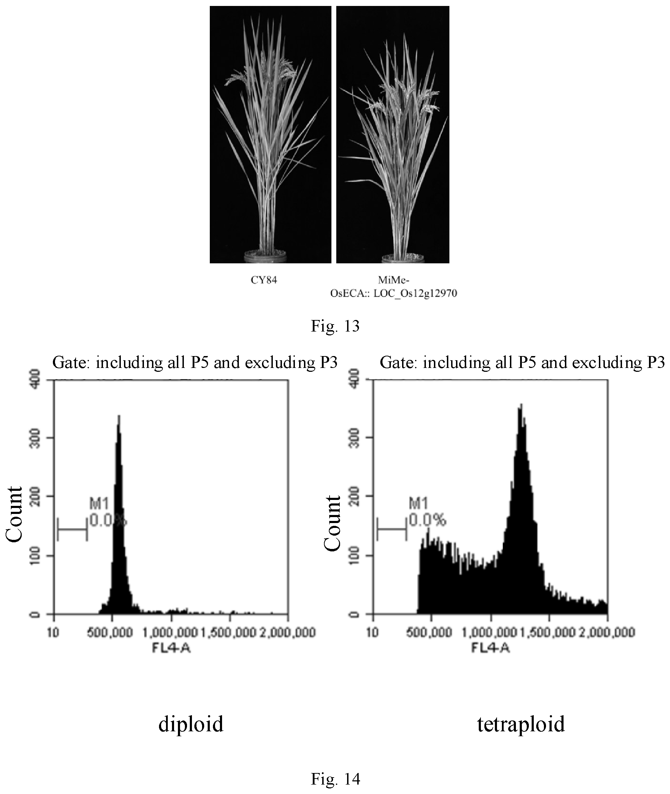

The obtained PCR fragments were sequenced to select lines homozygous for all three MiMe genes. The genotypes of the obtained lines are shown in Fig. 12, and the plant phenotypes are shown in Fig. 13.

### 4) MiMe-OsECA::LOC _Os12g12970 produces clonal diploid progeny

In rice, homozygous MiMe mutants are tetraploid. If MiMe-OsECA::LOC _Os12g12970 produces clonal diploid progeny, then a certain proportion of diploid plants can be detected in its progeny. T1 progeny seeds obtained by selfing each MiMe-OsECA::LOC_Os12g12970 line were germinated, and progeny ploidy was analyzed by flow cytometry at the seedling stage. As shown in Fig. 14, in progeny of MiMe-OsECA::LOC_Os12g12970, in addition to tetraploids, diploids were also detected, indicating that MiMe-OsECA::LOC_Os12g12970 indeed produced diploid progeny.

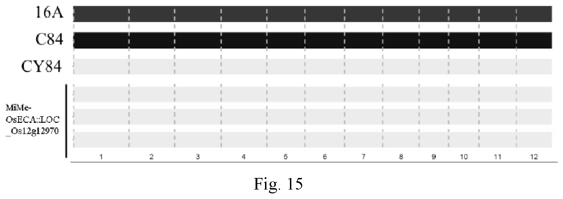

Whole-genome sequencing was performed on the obtained diploid progeny of MiMe-OsECA::LOC _Os12g12970, and it was found, as shown in Fig. 15, that their genotypes were completely identical to that of the control parent CY84 and exhibited genome-wide heterozygosity. This indicates that the LOC_Os12g12970 gene can be effectively used for synthetic apomixis.

### Example 5: Gene editing of the promoter region of the endogenous rice gene LOC_Os12g1297 can induce production of haploid progeny

The expression pattern of a gene is controlled by its transcriptional regulatory sequences. Therefore, modification of the promoter of an endogenous gene can achieve changes in the expression pattern and expression level of the endogenous gene. To modify the endogenous rice gene LOC_Os12g1297 so that it is expressed in egg cells, thereby achieving an effect similar to ectopic expression of this gene for producing haploids, the following was carried out.

### 1) Designing gRNAs targeting the LOC_Os12g1297 gene

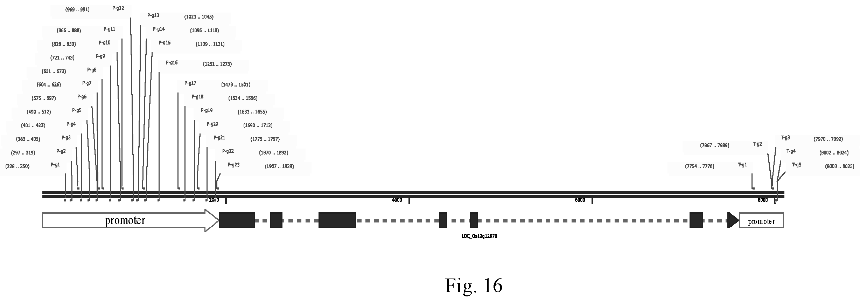

Several gene-editing knockout target sites were uniformly designed for possible regulatory sequences about 2000 bp upstream and about 500 bp downstream of the LOC_Os12g1297 gene. The specific sequences of the designed target sites and their approximate positions on the gene are shown in Table 2 and Fig. 16.

**Table 2. Design of editing target sites in regulatory sequences of the LOC_Os12g1297 gene**

| Target name | Target sequence |
|---|---|
| P-g1 | CAAGTCGACGACAACTAGAT AGG |
| P-g2 | TCTCGATGAGTTCGTACTCG AGG |
| P-g3 | TTATCTCTGATGTCGATGCC TGG |
| P-g4 | GAACAAACCAATACACCGCC AGG |
| P-g5 | TCCCTAGTTCAACTTAGATA TGG |
| P-g6 | TATAGAAGGAATTCCGAATA AGG |
| P-g7 | GTATGGTTCCGTATAAGACT TGG |
| P-g8 | GTCGATTACTATTGGGTATG TGG |
| P-g9 | AGGCGCATCATGTTAGTACG AGG |
| P-g10 | TTCATCCAGCACAAAGGCAG CGG |
| P-g11 | GGCACCATCCGTTCAAGATA GGG |
| P-g12 | AACATACACAACACATACAT AGG |
| P-g13 | TTGCAAGTCCAATGAAGAAT TGG |
| P-g14 | TGAAATGTGTGAGCCATTCC GGG |
| P-g15 | AATCCCCAGAATTCCCGGAA TGG |
| P-g16 | TCACAAAGATTTGTGTGTAT AGG |
| P-g17 | TTGAGGTTAACACTACACAT AGG |
| P-g18 | ACAAATATCACCAAAGTCAC AGG |
| P-g19 | GTATTAGGACTGATAAAGTA GGG |
| P-g20 | CAGTAAGGATCTCCGTTAAC GGG |
| P-g21 | AGATGTGTGTAGAGATGAGA AGG |
| P-g22 | ATCAAGGGCGGCCGGAACGG CGG |
| P-g23 | GATCGAAGCGGCCGTCAGGC TGG |
| T-g1 | GGTGTGTCTGCCATGTGGGG TGG |
| T-g2 | GTAGTGTGCCAATCTGGCAC AGG |
| T-g3 | GTGTGCCAATCTGGCACAGG TGG |
| T-g4 | ACCGACCAACCCTGTCCAAT GGG |
| T-g5 | AACCGACCAACCCTGTCCAA TGG |

Subsequently, for each target site, two complementary DNA sequences were designed respectively as follows: GGCA was added before the forward sequence, and AAAC was added before the reverse complementary sequence.

SK-gRNA has two AarI restriction sites. After digestion with AarI, a vector having sticky ends was formed. After denaturation and annealing of the forward and reverse primers of the designed target sequence, the target sequence was ligated into the previously constructed intermediate vector SK-gRNA using T4 ligase to form a single target gRNA.

### 2) Tandem assembly of multiple gRNAs and construction of the final binary expression vector

Taking advantage of the fact that BglII and BamHI, NheI and XbaI, and SalI and XhoI are isocaudomers, gRNAs were assembled onto the pC1300-Cas9 vector to obtain vectors simultaneously containing multiple editing target sites: pC1300-Cas9-Promoter-g1-g5, pC1300-Cas9-Promoter-g6-g10, pC1300-Cas9-Promoter-g11-g15, pC1300-Cas9-Promoter-g16-g20, pC1300-Cas9-Promoter-g21-g23, pC1300-Cas9-Terminator-g1-g4, and the like, for subsequent genetic transformation.

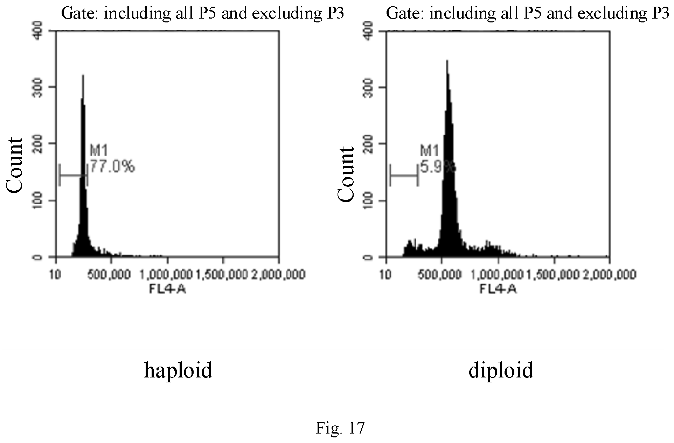

The specific transformation method was as follows: gene editing vectors pC1300-Cas9-Promoter-g1-g5, pC1300-Cas9-Promoter-g6-g10, pC1300-Cas9-Promoter-g11-g15, pC1300-Cas9-Promoter-g16-g20, pC1300-Cas9-Promoter-g21-g23, and pC1300-Cas9-Terminator-g1-g4 were introduced into Agrobacterium tumefaciens strain EHA105 by electroporation, and this binary expression vector was introduced into calli of rice Chunyou 84 by an Agrobacterium-mediated method. The specific transformation method was as follows: embryos of hybrid rice Chunyou 84 seeds were sterilized and then inoculated onto a medium for inducing callus tissue. After culture for 1 week, embryogenic calli that were vigorous, light yellow in color, and relatively loose were selected as transformation recipients. Rice calli were respectively infected with EHA105 strains containing the target plasmids, cultured in the dark at 25°C for 3 days, and then resistant calli and transgenic plants were screened on a selection medium containing 50 mg/L hygromycin. Transgenic plants growing normally on the hygromycin selection medium were selected. PCR detection was performed on the obtained lines to screen multiple lines in which the LOC_Os12g1297 promoter region and terminator region were edited, and flow cytometric screening was performed on their progeny to identify whether haploid progeny were produced (Fig. 17). The statistical results of haploid detection are shown in Table 3.

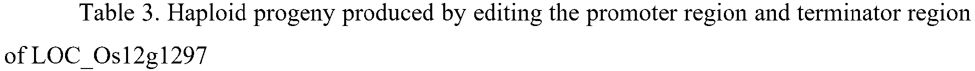

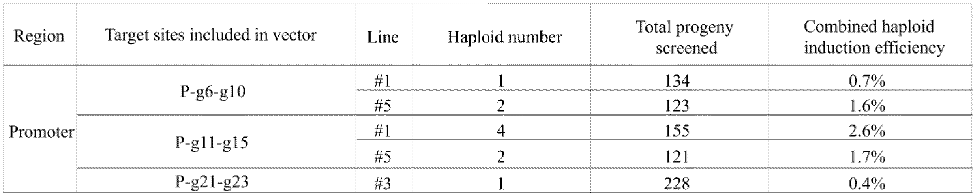

Based on the above results, modification of the regulatory sequences of the identified endogenous gene can also induce production of haploids.

### Example 6

### I. Verification of endogenous transcripts of LOC_Os12g12970

LOC_Os12g12970 is mainly expressed in sperm cells, that is, in mature pollen. First, primers for amplifying endogenous fragments of the full-length LOC_Os12g12970 transcript were designed: LOC_Osl2g12970-F: ATGGCGGGCGACGGCGGCAAC; LOC_Os12g12970-R: TTATTGATCAAGACCAGCAACTATCCTTCTCC.

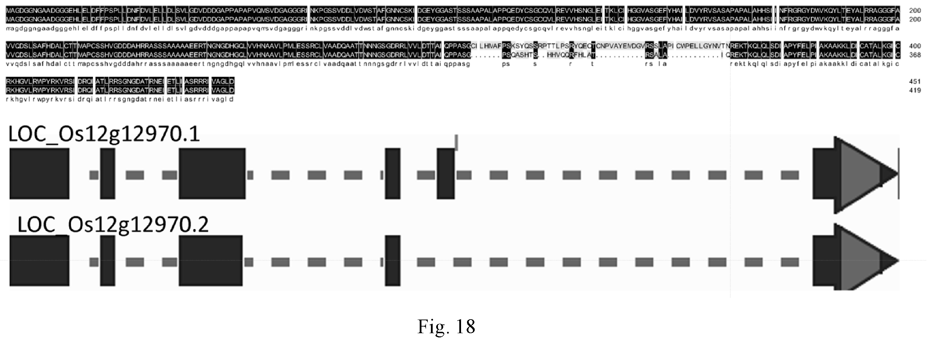

Subsequently, RNA was extracted from mature rice pollen and reverse-transcribed into cDNA as a template. The full-length CDS sequence was amplified by PCR and subjected to Sanger sequencing, and it was found that the main transcript form actually produced was LOC _Os12g12970.2 (SEQ ID NO:27 shows the LOC _Os12g12970.2 CDS sequence, SEQ ID NO:28 shows the LOC _Os12g1\2970.2 protein sequence, and SEQ ID NO:29 shows the LOC_Os12g12970-gDNA genomic gene sequence), and the specific differences between this transcript and the previously annotated splicing form and the formed protein are shown in Fig. 18. Therefore, LOC_Os12g12970 may have different splicing forms, including LOC _Os12g12970.2 and other splicing forms that have not yet been detected.

### II. Verification of effects of LOC_Os12g12970.2 and genomic fragments

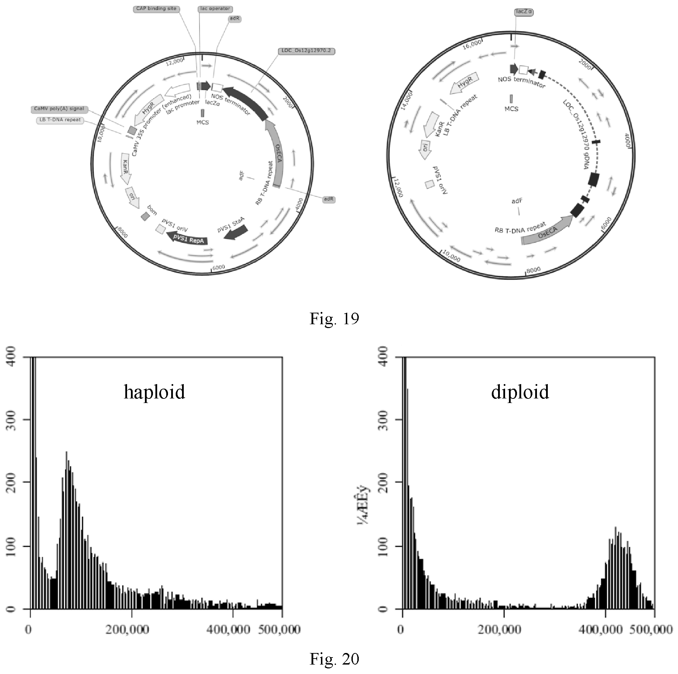

To verify whether the newly discovered splicing form LOC _Os12g12970.2 and other forms of products that may be produced from its genomic fragment also have the potential to induce parthenogenesis and produce haploids, an experiment was designed in which LOC _Os12g12970.2 and LOC _Os12g12970-gDNA were ectopically expressed in egg cells, and corresponding vectors were obtained (as shown in Fig. 19). Subsequently, the obtained vectors were transformed into hybrid rice CY84 by Agrobacterium-mediated transformation, and corresponding transgenic plants were obtained. Flow cytometric detection of the corresponding progeny (as described above) showed that both OsECA::LOC _Os12g12970.2 and OsECA::LOC _Os12g12970-gDNA could efficiently induce production of haploid progeny (Table 4).

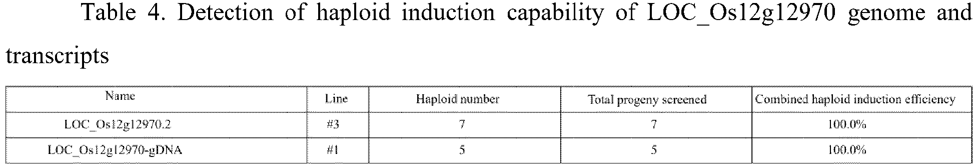

The results indicate that transcript LOC_Os12g12970.2 and the full genomic sequence thereof also have effects of inducing parthenogenesis and haploids.

### Example 7: Transient expression of LOC_Os12g12970 in an egg cell induces haploids

To determine whether transient expression of the LOC_Os12g12970 gene in an egg cell also has the capability to induce haploids, a carbon nanoparticle carrier was selected for transient expression of this gene in egg cells. The vector used was the same OsECA::LOC_Os12g12970-gDNA vector as described above in Fig. 19. The carbon nanoparticle encapsulation method was as follows: plasmid 10 ng/µL, carbon nanoparticles 1X, Tween-20 0.5%, 0.01 M MES pH 5.8. The mixed solution was then injected with a syringe near the ovary of hybrid rice CY84 before flowering, with multiple injections, and kept moist. Subsequently, the plants were allowed to grow normally until mature seed setting. After seed germination, flow cytometric analysis was used to detect whether haploids were produced. The results are shown in Fig. 20. Among 192 progeny injected with the OsECA::LOC_Os12g12970-gDNA vector, 3 haploid progeny were detected, with a ratio of about 1.6%.

## Claims

1. A protein having parthenogenesis-inducing capability, wherein the protein is a transcription factor of the RWP-RK protein family, and comprises the following conserved domains: TITFEDIRKYFHLPI and KEAAKELNVCLTTLKKICREHGIPRWPHRKIKSLNKLIKNI; and wherein the protein comprises a sequence having greater than 60% homology to SEQ ID NO:1 or SEQ ID NO:2, preferably greater than 70%, 80%, or 90% homology, more preferably greater than 95%, 98%, or 99% homology, and still has parthenogenesis-inducing capability.

2. The protein of claim 1, wherein the protein is LOC_Os12g12970 (SEQ ID NO:4), LOC _Os01g14420 (SEQ ID NO:6), LOC_Os04g47640, or LOC _Os12g12970.2 (SEQ ID NO:28) in rice; homologous proteins in maize are GRMZM2G004663 (SEQ ID NO:8) and AC233880 (SEQ ID NO:10); homologous proteins in wheat are TRAES3BF003800040CFD (SEQ ID NO:12) and TraesCS7A03G1206100 (SEQ ID NO:14); homologous proteins in sorghum are Sobic.003G000800 (SEQ ID NO:16) and Sobic.008G082000 (SEQ ID NO:18); a homologous protein in soybean is Glyma.06G198300 (SEQ ID NO:20); a homologous protein in foxtail millet is Seita.3G091700 (SEQ ID NO:22); homologous proteins in tomato are Solyc08g062200 (SEQ ID NO:24) and Solyc12g011190 (SEQ ID NO:26); or the protein comprises a homologous sequence having greater than 22% homology to LOC _Os12g12970, preferably greater than 35% homology, more preferably greater than 45% homology; or comprises a homologous sequence having greater than 42% homology to LOC_Os01g14420, preferably greater than 60% homology, more preferably greater than 70% homology; or comprises a homologous sequence having greater than 60% homology to any of the foregoing proteins, preferably greater than 70%, 80%, or 90% homology, more preferably greater than 95%, 98%, or 99% homology, and still has parthenogenesis-inducing capability.

3. A gene encoding the protein of claim 1 or 2, preferably wherein the gene is LOC_Os12g12970 (SEQ ID NO:3), LOC_Os01g14420 (SEQ ID NO:5), LOC_Os04g47640, LOC _Os12g12970.2 (SEQ ID NO:27), or the genomic gene shown in SEQ ID NO:29 in rice; homologous genes in maize are GRMZM2G004663 (SEQ ID NO:7) and AC233880 (SEQ ID NO:9); homologous genes in wheat are TRAES3BF003800040CFD (SEQ ID NO:11) and TraesCS7A03G1206100 (SEQ ID NO:13); homologous genes in sorghum are Sobic.003G000800 (SEQ ID NO:15) and Sobic.008G082000 (SEQ ID NO:17); a homologous gene in soybean is Glyma.06G198300 (SEQ ID NO:19); a homologous gene in foxtail millet is Seita.3G091700 (SEQ ID NO:21); and homologous genes in tomato are Solyc08g062200 (SEQ ID NO:23) and Solyc12g011190 (SEQ ID NO:25).

4. Use of the protein of claim 1 or 2, or the gene of claim 3, for inducing parthenogenesis in a plant, specifically for inducing haploids; or use thereof in apomixis.

5. The use of claim 4, wherein the plant is a monocotyledonous plant or a dicotyledonous plant. The monocotyledonous plant is preferably a plant of Poaceae, including but not limited to rice (*Oryza sativa*), maize (*Zea mays*), wheat (*Triticum aestivum*), barley (*Hordeum vulgare*), rye (*Secale cereale*), oat (*Avena sativa*), sorghum (*Sorghum bicolor*), proso millet (*Panicum miliaceum*), foxtail millet (*Setaria italica*), sugarcane (*Saccharum officinarum*), perennial ryegrass (*Lolium perenne),* annual ryegrass (*Lolium multiflorum*)*,* fescue *(Festuca arundinacea),* wildrye (*Elymus spp*.), crested wheatgrass (*Agropyron cristatum*), bermudagrass (*Cynodon dactylon*), zoysiagrass (*Zoysia japonica*), tall fescue *(Festuca arundinacea),* bromegrass (*Bromus inermis*), sudangrass (*Sorghum sudanense*), and reed (*Phragmites australis*). The dicotyledonous plant preferably includes plants of the following families and genera: Fabaceae, such as soybean (*Glycine max*), peanut (*Arachis hypogaea*), alfalfa (*Medicago sativa*), cowpea (*Vigna unguiculata*), mung bean (*Vigna radiata*), adzuki bean (*Vigna angularis*), chickpea (*Cicer arietinum*), hyacinth bean (*Lablab purpureus*), red clover (*Trifolium pratense*), white clover (*Trifolium repens*), broad bean (*Vicia faba*), and lupin (*Lupinus albus, Lupinus angustifolius*); Brassicaceae, such as rapeseed (*Brassica napus*), Chinese cabbage (*Brassica rapa subsp. pekinensis*), cabbage *(Brassica oleracea* var. *capitata*), radish (*Raphanus sativus*), mustard (*Brassica juncea*), Chinese kale (*Brassica oleracea* var. *alboglabra*), kale (*Brassica oleracea* var. *sabellica*), turnip (*Brassica rapa subsp. rapa),* broccoli *(Brassica oleracea* var. *italica*), and cauliflower *(Brassica oleracea* var. *botrytis);* Solanaceae, such as potato (*Solanum tuberosum*), tomato (*Solanum lycopersicum*), chili pepper *(Capsicum annuum),* eggplant (*Solanum melongena*), tobacco (*Nicotiana tabacum*), goji berry (*Lycium barbarum*), Chinese lantern plant (*Physalis alkekengi*), black nightshade (*Solanum nigrum*), sweet potato (*Ipomoea batatas*), green pepper *(Capsicum annuum),* and bird pepper (*Capsicum frutescens*); Cucurbitaceae, such as cucumber (*Cucumis sativus*), watermelon (*Citrullus lanatus*), melon (*Cucumis melo),* pumpkin *(Cucurbita spp.,* for example C. *pepo, C. maxima,* and C. *moschata),* loofah *(Luffa cylindrica),* bitter melon *(Momordica charantia),* wax gourd *(Benincasa hispida),* bottle gourd *(Lagenaria siceraria),* chayote *(Sechium edule),* and zucchini *(Cucurbita pepo);* Asteraceae, such as sunflower *(Helianthus annuus),* lettuce *(Lactuca sativa),* dandelion *(Taraxacum officinale),* garland chrysanthemum *(Chrysanthemum coronarium),* and asparagus lettuce *(Lactuca sativa* var. *angustana);* Apiaceae, such as carrot *(Daucus carota subsp. sativus),* celery *(Apium graveolens),* coriander/cilantro *(Coriandrum sativum),* fennel *(Foeniculum vulgare),* and dill *(Anethum graveolens);* Vitaceae, such as grape *(Vitis vinifera),* Amur grape *(Vitis amurensis),* fox grape *(Vitis labrusca),* and Eurasian grape *(Vitis vinifera ssp. sativa);* Malvaceae, such as cotton *(Gossypium hirsutum);* Pedaliaceae, such as sesame *(Sesamum indicum)* and African sesame *(Sesamum alatum);* Liliaceae, such as onion *(Allium cepa),* garlic *(Allium sativum),* Chinese chive *(Allium tuberosum),* Welsh onion *(Alliumfistulosum),* lily *(Lilium spp.),* and daylily *(Hemerocallis citrina);* Amaranthaceae, such as spinach *(Spinacia oleracea),* Swiss chard *(Beta vulgaris* var. *cicla),* amaranth *(Amaranthus tricolor),* and *Amaranthus spp.;* Rosaceae, such as strawberry *(Fragaria* × *ananassa);* and plants of other families and genera, such as beet *(Beta vulgaris, Chenopodiaceae),* cassava *(Manihot esculenta, Euphorbiaceae),* coffee *(Coffea arabica, Rubiaceae),* cacao *(Theobroma cacao, Sterculiaceae),* and castor bean *(Ricinus communis, Euphorbiaceae).*

6. A method for inducing a plant haploid, wherein the method is implemented by expressing, in an egg cell, a gene encoding the protein of claim 1 or 2, and subsequently identifying and obtaining haploid progeny from progeny thereof by flow cytometric screening; or by combining the above haploid induction method with a clonal gamete method, such as simultaneously mutating OSD1, PAIR1, and REC8 in rice, and subsequently obtaining clonal progeny plants from progeny thereof.

7. The method of claim 6, wherein expression of the gene is driven by a heterologous or homologous promoter active in an egg cell.

8. The method of claim 7, wherein the promoter is selected from AtDD45 (At2g21740), AtEC1.1 (Atlg76750), AtEC1.3 (At2g21750), AtEC1.4 (At4g39340), AtEC1.5 (At5g64720), OsECA1 (LOC_Os03g18530), OsECA2 (LOC_Os11g06730), OsECA3 (LOC_Os12g06970), or another heterologous or homologous promoter having egg cell expression characteristics;
specifically, the promoter of an endogenous gene encoding the protein is modified by gene editing or mutagenesis so that the endogenous gene is expressed in an egg cell; or an activation system is expressed in an egg cell to induce expression of the endogenous gene in the egg cell, for example, the activation system is a CRISPR/Cas9-based specific gene activation system CRISPRa, such as dCas9-TV, dzCas9-Act3.0, dSpRY-Act3.0, or the like.

9. The method of claim 6, wherein an encoding sequence of the protein is inserted downstream of an endogenous promoter active in an egg cell to induce expression of the gene in an egg cell; or a genome is modified to achieve large-fragment deletion and chromosomal inversion, so as to recombine the endogenous gene with a promoter on the genome to induce expression of the gene in an egg cell.

10. The method of claim 6, wherein the protein is expressed by transient expression, particularly wherein the expression site is an egg cell. For example, the transient expression is carried out by nanoparticle-mediated delivery of a plasmid.

11. The method of any one of claims 6 to 10, wherein haploid plants are obtained from progeny of the resulting plant material by flow cytometric screening, molecular markers, phenotypic identification, or marker-assisted methods, and subsequently the haploid plants may be subjected to chemical treatment or spontaneous genome doubling events to obtain genotype-homozygous doubled haploid materials for use in plant breeding processes;
in addition, when combined with clonal gamete technology, clonal seeds and plants are obtained from progeny thereof, thereby achieving apomixis in plants, which may further be used for hybrid plant breeding or fixation of plant heterozygous genotypes, and for production of hybrids.
